# EUROPEAN PATENT SPECIFICATION

(11) **EP 3 584 323 B1**
(45) Date of publication and mention of the grant of the patent: **17.03.2021**
(21) Application number: 19187738.0
(22) Date of filing: 10.12.2013
(51) Int. Cl.: C12P 7/40, C12P 7/56, C12P 13/08, C12M 1/34

(54) **BATCH FEED PROCESS FOR FERMENTING SUGARS**
CHARGENZUFUHRPROZESS ZUR FERMENTIERUNG VON ZUCKERN
PROCÉDÉ D'ALIMENTATION DISCONTINUE POUR FERMENTATION DE SUCRES

(30) Priority: 10.12.2012 US 201261735448 P
(43) Date of publication of application: 25.12.2019
(62) Divisional of application: 13862320.2
(73) Proprietor: Cargill, Incorporated, Wayzata, MN 55391 (US)
(72) Inventor: GOKARN, Ravi, Omaha, Nebraska 68130 (US); PARSONS, Matthew, Blair, Nebraska 68008 (US); WALCKER, Derran, Omaha, Nebraska 68134 (US); SPENCER, Joseph, Bennington, Nebraska 68007 (US)
(74) Representative: Forresters IP LLP

(56) References cited:
- EP-A1- 2 241 632
- WO-A2-2004/018645
- WO-A2-2007/057018
- US-A1- 2009 011 481
- SIEW LING HII ET AL: "Pullulanase: Role in Starch Hydrolysis and Potential Industrial Applications", ENZYME RESEARCH, vol. 2012, 921362, 12 June 2012 (2012-06-12), pages 1-14, XP055282385, DOI: 10.1155/2012/921362

## Description

### FIELD OF THE INVENTION

The present invention relates to a process for fermenting sugars. More particularly, the present invention relates to a batch feed process for fermenting sugars.

### BACKGROUND OF THE INVENTION

Fermentation processes are used commercially at large scale to produce organic molecules such as ethanol, citric acid and lactic acid. In those processes, a carbohydrate is fed to a organism that is capable of metabolizing it to the desired fermentation product. The carbohydrate and organism are selected together so that the organism is capable of efficiently digesting the carbohydrate to form the product that is desired in good yield. It is becoming more common to use genetically engineered organisms in these processes, in order to optimize yields and process variables, or to enable particular carbohydrates to be metabolized.

Starch is a widely available and inexpensive carbohydrate source. It is available from a wide variety of plant sources such as corn, wheat, rice, barley, and the like. Many organisms are not capable of metabolizing starch directly, or else metabolize it slowly and inefficiently. Accordingly, it is common to treat starch before feeding it into the fermentation process, in order to break it down into monosaccharides that the organism can ferment easily.

Usually, starch is hydrolyzed to form a mixture containing mainly glucose (i.e., dextrose). However, complete hydrolysis to glucose adds significant cost, so most commercially available glucose products tend to contain a small amount of fructose and various oligomeric polysaccharides. Unfortunately, many organisms cannot metabolize the oligomers, either, and so these carbohydrate values are wasted.

The published patent application US2009/011481 discloses a fermentation process for fermenting a starch hydrolysate, which comprises a step of adding to the fermentation broth an effective quantity of at least one enzyme that depolymerises at least one oligomeric saccharide to glucose and also discloses transglucosidase enzymes which convert isomaltose into glucose.

### SUMMARY OF THE INVENTION

Isomaltose is a component that may be formed during the process of preparing starch hydrolysate raw materials having high glucose concentrations. It has been found that isomaltose is a particularly undesirable component to be present, particularly at the later stages of the present process to ferment glucose from a starch hydrolysate source to form fermentation products. While not being bound by theory, it is believed that isomaltose may interfere with the product yield not only by preventing fermentation of the two constituent glucose monomer components of the isomaltose, but also by reacting with the final fermentation products. The isomaltose may react with the fermentation products to form for example ester and other chemicals, which are formed via the reaction of a plurality of available hydroxy functionalities of the isomaltose. For example, if the product is an amino acid, the isomaltose may react with the product via the Maillard reaction to produce chemical compounds which cause browning. Thus each isomaltose compound present at the end of the fermentation process has a multiplier effect in reducing the yield and/or quality of the desired final fermentation product.

It has further been found that addition of an enzyme to a starch hydrolysate material having high glucose concentration tends to render the enzyme less effective. The present invention provides an efficient and elegant way to ferment starch hydrolysates having high glucose concentration. The present process is particularly beneficial for the preparation of fermentation products to be used in non-food applications. Surprisingly fermentation products used in non-food applications require a different standard of purity, because some residual ingredients of fermentation processes that may be present in food products are not acceptable in non-food applications.

In a first embodiment, a batch fermentation process is provided that ferments a starch hydrolysate containing 80-98 weight percent of glucose based on total carbohydrate and 0.3-5% weight percent of isomaltose based on total carbohydrate to a fermentation produc, wherein the fermentation product comprises an organic acid selected from the group consisting of hydroxyl carboxylic acids, dicarboxylic acids, and tricarboxylic acids; or wherein the fermentation product is selected from the group consisting of lactic acid, citric acid, malonic acid, hydroxy butyric acid, adipic acid, lysine, ketoglutaric acid, glutaric acid, 3-hydroxy-proprionic acid, succinic acid, malic acid, fumaric acid, itaconic acid, muconic acid, methacrylic acid, and acetic acid

. In this process, a fermentation broth is formed containing a first portion of a total amount of the starch hydrolysate so that the fermentation broth has an initial glucose concentration of at least 50 g/L. The starch hydrolysate in the fermentation broth is fermented in an initial fermentation step to produce a fermentation product until the fermentation broth contains 30 g/L or less of glucose. After achieving this glucose concentration, an effective amount of at least one active enzyme that converts isomaltose into glucose is added to the fermentation broth. Then the remaining portion of the total amount of starch hydrolysate containing 80-98 weight percent of glucose is fed into the fermentation broth to maintain a glucose concentration of from 5 to 15 g/L in the fermentation broth throughout the feeding step. The final fermentation broth containing the fermentation product is then produced.

### DETAILED DESCRIPTION OF PRESENTLY PREFERRED EMBODIMENTS

The embodiments of the present invention described below are not intended to be exhaustive or to limit the invention to the precise forms disclosed in the following detailed description. Rather a purpose of the embodiments chosen and described is so that the appreciation and understanding by others skilled in the art of the principles and practices of the present invention can be facilitated.

In the present batch fermentation process, a starch hydrolysate is used as the starting material. The starch may be obtained from any suitable starch source, such as corn, wheat, rice, barley, potatoes, cassava, and the like. The starch hydrolysates used in the present fermentation process are preferably obtained from a starch that has undergone a liquefaction and saccharification in order to obtain a high concentration of glucose.. The balance of carbohydrate in such hydrolysates are composed of varying concentrations of fructose, maltose, isomaltose, panose, and other DP3 and DP4 (i.e. oligomers of glucose) or greater glucose oligomers. Such starch hydrolysates are a particularly preferred fermentation substrate for use in this invention. In the present process, the starch hydrolysate to be used in the fermentation process contains 80-98 weight percent of glucose based on total carbohydrate and 0.3-5 weight percent of isomaltose based on total carbohydrate (for example, from 0.4-5 weight percent, from 0.5-3 weight percent, and from 0.7-2.5 weight percent isomaltose based on total carbohydrate). Optionally, the starch hydrolysate to be used in the fermentation process contains 85-98 weight percent of glucose based on total carbohydrate or 90-97 wt% of glucose based on the total carbohydrate. Optionally, the starch hydrolysate to be used in the fermentation process contains 0.5-3% weight percent of isomaltose based on total carbohydrate (for example, from 0.4-5 weight percent, from 0.5-3 weight percent, and from 0.7-2.5 weight percent isomaltose based on total carbohydrate), or in some instances where higher level of isomaltose are present from 1-2 percent by weight isomaltose based on the total carbohydrate. Optionally, at least about 90% by weight of the solids content of the starch hydrolysate is carbohydrate. Optionally, at least about 95% by weight of the solids content of the starch hydrolysate is carbohydrate. Typically at least about 97% by weight of solids content (for example at least about 98 % by weight of solids content of the starch hydrolysate is carbohydrate) and in some instances at least 99% by weight of solids content of the starch hydrolysate is carbohydrate. Optionally, the starch hydrolysate is provided as a starting material composition having a dry solids content of from about 20 to about 70%. Optionally, the starch hydrolysate is provided as a starting material composition having a dry solids content of from about 25 to about 45%. Optionally, the starch hydrolysate is provided as a starting material composition having a dry solids content of from about 50 to about 70%. Starch hydrolysates having higher solids content can be easier and less expensive to ship than those which have lower solids content, which provides particular benefit for applications where the facility for carrying out the starch hydrolysis is some distance away from the facility for carrying out the batch fermentation process.

A fermentation broth is formed containing a first portion of a total amount of the starch hydrolysate so that the fermentation broth has an initial glucose concentration of at least 50 g/L. The fermentation broth is prepared by mixing the starch hydrolysate, water, and nutrients necessary to support growth of the fermentation organism and inoculating with an organism suitable for carrying out the desired fermentation. In an embodiment, the initial glucose concentration is from about 50 g/L to about 170 g/L. In an embodiment, the initial glucose concentration is from about 70 g/L to about 140 g/L. In an embodiment, the initial glucose concentration is from about 80 g/L to about 130 g/L. In an embodiment, the initial glucose concentration is from about 90 g/L to about 120 g/L.

Embodiments using a higher initial concentration of glucose in the fermentation broth are particularly advantageous, because one may take advantage of the better economics that come with using higher starting concentrations of starting materials.

The organism used in the present fermentation is one which can ferment glucose to the desired fermentation product. As such, the particular organism used is selected in relation to the fermentation product that is desired. The organism may be naturally occurring, or may be a mutant or recombinant strain. Examples of organisms include various species of bacilli, lactobacilli, filamentous fungi, and yeast, including wild-types and mutated or recombinant types. Suitable organisms useful for producing lactic acid include wild-type bacteria from the genera Lactobacillus, Pediococcus, Lactococcus and Streptococcus and wild-type fungi of the genera Rhizopus. In addition, recombinant yeast strains of the genera Kluyveromyces, Pichia, Hansenula, Candida, Trichosporon, Issatchenkia, or Yamadazyma, having exogenous LDH genes are suitable. Such recombinant yeast strains are described in WO 99/14335, WO 00/71738 and WO 02/42471, for example. Additional organisms include fungi, algae and archaea. Mixtures of organisms are also specifically contemplated.

The fermentation broth will also include water and preferably includes nutrients, such as proteins (or other nitrogen source), vitamins and salts, which are necessary or desirable for cell function. Other components may also be present in the fermentation broth, such as buffering agents, fermentation products (which tend to accumulate as the fermentation progresses), and other metabolites. In cases where the fermentation is an acid, it is common to buffer the broth with a base such as calcium hydroxide or calcium carbonate, ammonia or ammonium hydroxide, sodium hydroxide, or potasium hydroxide in order to maintain a pH at which the organism functions well.

The present batch fermentation process adds starch hydrolysate at two separate times in the fermentation process in order to ferment a total amount of starch hydrolysate. The first portion is added before fermentation is conducted to reach the desired glucose concentration and addition of the enzyme. The first portion of the total amount of the starch hydrolysate preferably contains from about 50 to about 80 percent of the total amount of starch hydrolysate to be added to the fermentation broth.

The starch hydrolysate is then fermented in the fermentation broth in an initial fermentation step to produce a fermentation product until the fermentation broth contains 30 g/L or less of glucose. In an embodiment, the starch hydrolysate is fermented until the fermentation broth contains about 25 g/L or less of glucose in the initial fermentation step. In an embodiment, the starch hydrolysate is fermented until the fermentation broth contains about 20 g/L or less of glucose. In an embodiment, the starch hydrolysate is fermented until the fermentation broth contains about 15 g/L or less of glucose. In some embodiments, a minimum amount of glucose is desirable in the system to assure that the fermentation process can continue to operate without interruption during the fermentation process. Thus, in some embodiments, the starch hydrolysate is fermented until the fermentation broth contains from 30 g/L to about 5 g/L of glucose, or from about 25 g/L to about 5 g/L of glucose, or from about 20 g/L to about 5 g/L of glucose, or from about 15 g/L to about 5 g/L of glucose. Similarly, in some embodiments, the starch hydrolysate is fermented until the fermentation broth contains from 30 g/L to about 8 g/L of glucose, or from about 25 g/L to about 8 g/L of glucose, or from about 20 g/L to about 8 g/L of glucose, or from about 15 g/L to about 8 g/L of glucose. Similarly, in some embodiments, the starch hydrolysate is fermented until the fermentation broth contains from 30 g/L to about 10 g/L of glucose, or from about 25 g/L to about 10 g/L of glucose, or from about 20 g/L to about 10 g/L of glucose, or from about 15 g/L to about 10 g/L of glucose.

The fermentation is carried out under conditions so that fermentation can occur. Although conditions can vary depending on the particular organism and desired fermentation product, typical conditions include a temperature of from about 20°C., preferably from about 30°C. to about 50°C., more preferably about 45°C. Usually the reaction mixture is mixed. The mixing may occur by sparging gas to the fermentation broth or alternatively via direct mechanical agitation or by other means.

Once this above described concentration of glucose is achieved during the initial fermentation step, an effective amount of at least one active enzyme that converts isomaltose into glucose is adding to the fermentation broth. The enzyme may be any enzyme effective for depolymerizing isomers having a 1,6 ether linkage. A preferred enzyme for acting on isomers having 1,6 linkages is trans-glucosidase.

The amount of enzyme to be used depends somewhat on the particular enzyme, the desired rate of reaction, and presence of any other oligomeric polysaccharide(s) that may also be effectively depolymerized by the enzyme. Enzyme activity is commonly measured in Units (U) of activity. One unit of enzyme activity is defined as the amount of enzyme activity that will convert one micro-mole of substrate per minute. In an embodiment, the enzyme is used in a quantity sufficient to provide about 0.05- 5 Units of enzyme activity for an isomaltose substrate per liter of fermentation broth. In another embodiment, the quantity of enzyme is from about 0.1- 3 Units of enzyme activity per liter of fermentation broth, In another embodiment, the quantity of enzyme is from about about 0.3- 1 Units of enzyme activity per liter of fermentation broth Similarly, these Units of enzyme activity per liter of fermentation broth may be used to determine the amount of enzyme suitable for conversion of other oligomeric polysaccharide(s) that may be present in the fermentation broth. The above number of Units is used to provide a good approximation of the amount of enzyme to be added to the fermentation broth, which is evaluated by experimental observation to determine actual amounts of enzyme required under conditions of use (e.g. temperature, pH and other conditions particular to a given fermentation process) to achieve the desired concentrations of residual oligosaccharides in the final fermentation broth at the lowest cost of enzymes.

The step of adding to the fermentation broth an effective amount of at least one active enzyme that converts isomaltose into glucose is carried out under conditions typically to obtain the conversion of the isomaltose and the fermentation of the glucose occur simultaneously. In an embodiment, the active enzyme is added to the fermentation broth in a single addition step. In an embodiment, the active enzyme is added to the fermentation broth in a single addition step metered over a time of from about 3 minutes to 3 hours. In an embodiment, the active enzyme is added to the fermentation broth in a plurality of addition steps after the initial fermentation step. In another embodiment, at least a portion of the enzyme is added after the remaining portion of the starch hydrolysate is fed, and the amount of isomaltose present in the fermentation broth is monitored and additional enzyme is added as needed to achieve the desired isomaltose level.

Addition of the enzyme after the initial fermentation step, once the glucose concentration level is achieved, maximizes its effectiveness, because it has been found that high glucose concentration compositions tend to experience reverse conversion of glucose to isomaltose and additionally that the enzyme itself is inhibited by high concentration of glucose. Further, the enzyme has a finite time of effectiveness, and so later addition to the fermentation process is advantageous to use the enzyme during its active lifetime.

In an embodiment, the total amount of starch hydrolysate additionally contains from about 1 to about 10 weight percent of maltose. This maltose preferably also is enzymatically converted into glucose. In an embodiment, the enzyme selected for addition is effective for conversion of both isomaltose and maltose into glucose. In another embodiment, an additional enzyme that is different from the isomaltose enzyme may also be introduced to convert maltose into glucose. The enzyme may be any enzyme that is more effective for depolymerizing isomers such as maltose having a 1,4 ether linkage. A preferred enzyme for acting on isomers having 1,4 linkages is glucoamylase. Optionally, the same or different enzymes may be introduced to covert other oligomeric polysaccharides that may be present in the starch hydrolysate into glucose.

The remaining portion of the total amount of starch hydrolysate containing 80-98 weight percent of glucose is fed into the fermentation broth batch to maintain a glucose concentration of from 5 to 15 g/L in the fermentation broth throughout the feeding step. It has been found that at concentrations higher than about 15 g/L, the isomaltose enzyme is more and more inhibited in activity as glucose concentration is increased.

In an embodiment of the present invention, the remaining portion of the total amount of starch hydrolysate added is provided as an addition composition having a solids content of from about 25 to about 70 weight percent. In an embodiment, this remaining portion is added as an addition composition having a solids content of from about 25 to about 45 weight percent, or as an addition composition having a solids content of from about 30 to about 40 weight percent, or as an addition composition having a solids content of from about 45 to about 70 weight percent.

The step of feeding the remaining portion of the total amount of starch hydrolysate into the fermentation broth is more preferably carried out to maintain a glucose concentration of from about 8 to about 12 g/L in the fermentation broth throughout the feeding step. This feeding step maximizes the amount of fermentation product that can be produced in a single batch operation, and minimizes the amount of enzyme required by most efficiently using the organisms and enzymes that are charged to the fermentation vessel.

In an embodiment of the present invention, the remaining portion of the total amount of starch hydrolysate is added in at least three sub-portion addition steps

In a preferred embodiment of the present invention, the remaining portion of the total amount of starch hydrolysate is added to the fermentation broth using a variable rate addition system. This system is preferred because it does not introduce temporarily high concentrations of glucose in the fermentation batch that would adversely affect the activity of the enzyme. Examples of such systems include a variable speed pump or a metering valve (such as a throttle valve) operably connected to a pump, which pump or valve can be utilized to vary the amount of hydrolysate introduced into the fermentation broth over time. In an embodiment, the starch hydrolysate addition is carried out over a time period of from about 4 to about 30 hours, for example from about 5 to about 24 hours, and in some cases to optimize product yield, enzyme usage and production costs, from about 10 to about 20 hours.

In an embodiment, during the addition of the remaining portion of the starch hydrolysate the glucose concentration is monitored by a real-time monitoring system. Real-time monitoring systems include systems that directly monitor glucose concentration and systems that indirectly monitor glucose concentration. Examples of real-time monitoring systems that typically directly monitor glucose concentration include systems based on infrared (IR) spectroscopy, near-infrared (NIR) spectroscopy systems, Fourier transform infrared (FTIR) systems, systems based on refractive index, automated enzyme based measurement systems such as a YSI 2950 Biochemistry Analyzer sold by YSI Life Sciences systems, high performance liquid chromatography (HPLC) based systems, gas chromatography (GC) based systems, and other real-time monitorying systems known to one of skill in the art. Additionally real-time monitoring systems that indirectly monitor/measure the glucose concentration of a fermentation process can be developed by determining the typical carbon distribution in a particular fermentation process and correlating the glucose concentration present in the fermentation broth to another parameter exhibited by the fermenation, such as, for example, a correlation of the glucose level present in the fermentation broth with a measurement of the carbon dioxide evolution rate and the amount of carbon dioxide present in an off-gas stream from the fermentation vessel. The carbon dioxide can be readily measured through use of a mass spectrometer or other suitable instrumental technique for measuring the components of the off-gas stream. In a preferred embodiment, the glucose concentration is monitored by a real-time monitoring system using infrared spectroscopy. In another preferred embodiment, the glucose concentration is monitored by a real-time monitoring system using near-infrared spectroscopy. In a particularly preferred aspect of the invention, the real time monitoring systems interface with equipment that controls the introduction of starch hydrolysate introduced into the fermentation broth to facilitate the maintenance of the glucose concentration in the fermentation broth at the desired concentration.

In another aspect of the invention, the glucose concentration is monitored by real-time monitoring system, and a control system utilizes the value of glucose concentration measured to control the introduction of starch hydrolysate (and optionally enzyme) into the fermentation broth. In this aspect of the invention, the starch hydrolysate utilized has greater than 60 percent by weight glucose concentration based on the total carbohydrate, typically greater than 70 percent by weight glucose concentration , for example, greater than 80 percent by weight glucose concentration based on the total carbohydrate and in many instances greater than 90 percent by weight glucose concentration based on the total carbohydrate.

The addition of the remaining portion of the starch hydrolysate is continued until the desired amount of starch hydrolysate has been added. After the desired amount of starch hydrolysate has been added, the fermentation is typically allowed to proceed until the final fermentation broth is produced. In a preferred embodiment, after the desired amount of starch hydrolysate has been added, the fermentation is allowed to proceed until the glucose concentration is at a concentration below 5 g/L, or preferably below 1 g/L, or more preferably below 0.5 g/L based on the volume of the final fermentation broth.

In an embodiment, the final fermentation broth comprises no more than about 3 g/L of isomaltose. In an embodiment, the final fermentation broth comprises no more than about 1 g/L of isomaltose, and preferably no more than about 0.5 g/L isomaltose.

The fermentation product comprises an organic acid selected from the group consisting of hydroxyl carboxylic acids, dicarboxylic acids, and tricarboxylic acids ; or wherein the fermentation product is selected from the group consisting of lactic acid, citric acid, malonic acid, hydroxy butyric acid, adipic acid, lysine, keto-glutaric acid, glutaric acid, 3-hydroxy-proprionic acid, succinic acid, malic acid, fumaric acid, itaconic acid, muconic acid, methacrylic acid, and acetic acid.

The activity of enzymes capable of converting isomaltose and other oligomeric polysaccharide(s) into glucose (such as transglucosidase (TG)), often is at least partially dependent on the pH and composition (including cation composition) of the fermentation broth being utilized. When the fermentation products comprise a carboxylic acid (including hydroxy carboxylic acid(s) and amino acids), it may be desirable to adjust the pH of the fermentation broth to enhance the activity of the enzyme. When desired, a basic compound typically would be added to the fermentation broth to adjust the pH of the broth and therefore enhance the activity of an enzyme, such as a TG enzyme. Basic compounds that may be utilized include compounds having the following cations: ammonium, sodium, potasium, and mixtures thereof.

When the fermentation process utilizes a gypsum recovery process, as described below, the preferred cation utilized comprises Calcium. In a gypsum recovery process, an organic acid fermetation product (including a hydroxy carboxylic acid) is made. During the fermentation, the pH of the fermentation broth is maintained at a desireable level using calcium hydroxide, often used in the form of lime. Once the fermenation is complete, it is desirable to enhance the recovery of the organic acid by adding sulfuric acid to acidulate the broth and thereby increase the percentage of acid that is recovered from the broth. The ions from the lime and sulfuric acid react to form calcium sulfate (gypsum), which can then be readily removed from the fermenation broth by precipitation.

Alternatively, ammonia or ammonium hydroxide is economical and abundant. Ammonium hydroxide has the advantage of providing not only pH buffering, but also supplies an additional bio-available nitrogen source to the fermentation. Examples of fermentations that employ ammonium hydroxide for pH control include fermentation processes for the manufacture of citric acid, lysine or other amino acids. Additionally, ammonium hydroxide is commonly employed in fermentation processes that typically incorporate the ammonium into the fermenation product (such as amino acids) or biomass (such as amino acids or citric acid and other processes known to one of skill in the art), can tolerate ammonium in the final product specifications or processes where the fermentation product is typically recovered through the use of ion exchange, crystallization or liquid-liquid extraction (such as citric acid, succinic acid, or fumaric acid, and other processes known to one of skill art).

Sodium hydroxide and potasium hydroxide are readily available. They typically would be utilized in fermentation processes that require only relatively minor pH control during the fermenation.

The fermentation product is recovered from the fermentation broth. The manner of accomplishing this will depend on the particular product. However, in general, the organism is separated from the liquid phase, typically via a filtration step or centrifugation step, and the product recovered via, for example, distillation, extraction, crystallization, membrane separation, osmosis, reverse osmosis, or other suitable technique. Organic acids typically will require that the fluid containing the organic acid (and salts thereof) be acidulated using acids such as sulfuric acid to recover the organic acid.

In an embodiment, the amount of enzyme that is used in the fermentation batch is substantially reduced as compared to a like process where all of the starch hydrolysate is charged to the fermentation broth at the beginning of fermentation and enzyme is added after the glucose concentration is reduced to a desired level (as described in US Patent Application Publication 2009/0011481). It has surprisingly been discovered that by holding back a portion of the total starch hydrolysate to be fermented until after the first portion is fermented to the desired glucose concentration level and enzyme is added, and then adding the remainder of the starch hydrolysate in manner to control the glucose concentration to a desired level, one can use substantially less enzyme overall. In an embodiment, the amount of enzyme used in the present batch process having portion of starch hydrolysate added after the enzyme is at least 25% less than the amount used in a like process having all of the starch hydrolysate added as an initial charge. In an embodiment, the amount of enzyme used in the present batch process having portion of starch hydrolysate added after the enzyme is at least 40% less than the amount used in a like process having all of the starch hydrolysate added as an initial charge. In an embodiment, the amount of enzyme used in the present batch process having portion of starch hydrolysate added after the enzyme is at least 50% less than the amount used in a like process having all of the starch hydrolysate added as an initial charge. In an embodiment of the present invention, the amount of enzyme to be added is calculated based on the amount of isomaltose in the total amount of starch hydrolysate and the activity of the enzyme when used in a fermentation broth that contains 20 g/L or less of glucose.

The present process provides the ability to make fermentation products on a production scale level with excellent yields and purity. In an embodiment, the batch process is carried out in to produce batches of at least 25,000 gallons of final fermentation broth.

The present disclosure describes specific embodiments in various aspects of the invention, such as preferred amounts of glucose content and enzyme addition conditions. Combinations of separately discussed aspects of the present invention with each other to provide preferred embodiments are specifically contemplated.

### EXAMPLES

Representative embodiments of the present invention will now be described with reference to the following examples that illustrate the principles and practice of the present invention.

The transglucosidase used is based on the TG-L2000 product available from DuPont Biosciences, with the following differences: 1) the final glycerol composition is less than 1%; 2) traces of NaCl are present in the product but <0.1%; 3) the following additional preservatives and stabilizers are added - Glucose (20-30% w/w) and sodium benzoate (0.2-0.4% w/w).

The fermentation broth consists of water as well as sufficient nutrients required to enable cell growth. The fermentation broth also contains an anti-foam agent to control foaming in the fermentation.

A fermentor is partially filled to 65% of volumetric operating capacity with the components of the fermentation broth as well as 60% of the total glucose to be added. The glucose source is a starch hydrolysate of which 95% of the carbohydrates are glucose. The temperature is controlled at a value that is suitable for the fermentation host organism, in this case 34° C. The fermentor is then inoculated with a suitable fermentation microorganism, in this case, a yeast engineered for the production of lactic acid. The pH is maintained above 3.1 by the addition of calcium hydroxide.

As the fermentation proceeds, the glucose is converted into lactic acid at a certain yield. The glucose concentration is monitored by a near infrared spectroscopy system (NIR) (Brueker - Matrix F model). When the glucose has been reduced to less than 20 g/L as measured by NIR, the transglucosidase enzyme as described above is added at a dose of 0.025% (volume enzyme solution/volume fermentation broth).

The fermentation is then allowed to further proceed until the glucose concentration reaches 10 g/L. The remaining 40% of the total glucose is then gradually added to the fermentor at a rate controlled to maintain the glucose between 8 g/L and 12 g/L as measured by the NIR.

Once 100% of the target dextrose has been added, the glucose feed is stopped and the fermentation proceeds until the glucose is reduced below 0.5 g/L.

Unless otherwise indicated, all parts and percentages are by weight and all molecular weights are weight average molecular weights.

## Claims

1. A batch fermentation process for fermenting a starch hydrolysate containing 80-98 weight percent of glucose based on total carbohydrate and 0.3-5% weight percent of isomaltose based on total carbohydrate to a fermentation product, comprising:
a) forming a fermentation broth containing a first portion of a total amount of the starch hydrolysate so that the fermentation broth has an initial glucose concentration of at least 50 g/L;
b) fermenting the starch hydrolysate in the fermentation broth in an initial fermentation step to produce a fermentation product until the fermentation broth contains 30 g/L or less of glucose;
c) adding to the fermentation broth an effective amount of at least one active enzyme that converts isomaltose into glucose;
d) feeding the remaining portion of the total amount of starch hydrolysate containing 80-98 weight percent of glucose based on total carbohydrate and 0.3-5% weight percent of isomaltose based on total carbohydrate into the fermentation broth to maintain a glucose concentration of from 5 to 15 g/L in the fermentation broth throughout the feeding step; and
e) producing a final fermentation broth containing the fermentation product;
wherein the fermentation product comprises an organic acid selected from the group consisting of hydroxyl carboxylic acids, dicarboxylic acids, and tricarboxylic acids; or
wherein the fermentation product is selected from the group consisting of lactic acid, citric acid, malonic acid, hydroxy butyric acid, adipic acid, lysine, keto-glutaric acid, glutaric acid, 3-hydroxy-proprionic acid, succinic acid, malic acid, fumaric acid, itaconic acid, muconic acid, methacrylic acid, and acetic acid.

2. The fermentation process of any of the preceding claims, wherein the fermentation product comprises lactic acid.

3. The fermentation process of any of the preceding claims, wherein the amount of enzyme to be added is calculated based on the amount of isomaltose in the total amount of starch hydrolysate and the activity of the enzyme when used in a fermentation broth that contains 20 g L or less of glucose.

4. The fermentation process of any of the preceding claims, wherein the glucose concentration of the fermentation broth containing a first portion of a total amount of the starch hydrolysate of step a) is from about 50 g/L to about 150 g/L.

5. The fermentation process of any of the preceding claims, wherein the starch hydrolysate contains 85-98 weight percent of glucose based on total carbohydrate.

6. The fermentation process of any of the preceding claims, wherein the starch hydrolysate contains 90-97% weight percent of glucose based on total carbohydrate.

7. The fermentation process of any of the preceding claims, wherein the starch hydrolysate contains 0.5-3% weight percent of isomaltose based on total carbohydrate.

8. The fermentation process of any of the preceding claims, wherein the starch hydrolysate contains 1-2% weight percent of isomaltose based on total carbohydrate.

9. The fermentation process of any of Claims 1-8, wherein a real-time monitoring system interfaces with equipment that controls the introduction of starch hydrolysate in step (d).

## Patentansprüche

1. Batch-Fermentationsverfahren zum Fermentieren eines Stärkehydrolysats, das 80 - 98 Gew.-% Glucose, basierend auf einem Gesamtkohlenhydrat, und 0,3 - 5 Gew.-% Isomaltose, basierend auf dem Gesamtkohlenhydrat, zu einem Fermentationserzeugnis enthält, umfassend:
a) Ausbilden einer Fermentationsbrühe, die einen ersten Anteil einer Gesamtmenge des Stärkehydrolysats enthält, sodass die Fermentationsbrühe eine Anfangsglucosekonzentration von wenigstens 50 g/l aufweist;
b) Fermentieren des Stärkehydrolysats in der Fermentationsbrühe in einem ersten Fermentationsschritt, um ein Fermentationserzeugnis zu erzeugen, bis die Fermentationsbrühe höchstens 30 g/l Glucose enthält;
c) Hinzufügen einer wirksamen Menge zu der Fermentationsbrühe von wenigstens einem aktiven Enzym, das Isomaltose in Glucose umwandelt;
d) Zuführen des verbleibenden Anteils an der Gesamtmenge des Stärkehydrolysats, die 80 - 98 Gew.-% Glucose, basierend auf dem Gesamtkohlenhydrat, und 0,3 - 5 Gew.-% Isomaltose enthält, basierend auf dem Gesamtkohlenhydrat, in die Fermentationsbrühe, um eine Glucosekonzentration von 5 bis 15 g/l in der Fermentationsbrühe während des Fütterungsschritts aufrechtzuerhalten; und
e) Erzeugen einer endgültigen Fermentationsbrühe, die das Fermentationserzeugnis enthält;
wobei das Fermentationserzeugnis eine organische Säure umfasst, die aus der Gruppe ausgewählt ist, die aus Hydroxylcarbonsäuren, Dicarbonsäuren und Tricarbonsäuren besteht; oder
wobei das Fermentationserzeugnis aus der Gruppe ausgewählt ist, die aus Milchsäure, Zitronensäure, Malonsäure, Hydroxybuttersäure, Adipinsäure, Lysin, Ketoglutarsäure, Glutarsäure, 3-Hydroxypropionsäure, Bernsteinsäure, Äpfelsäure, Fumarsäure, Itaconsäure, Muconsäure, Methacrylsäure und Essigsäure besteht.

2. Fermentationsverfahren nach einem der vorhergehenden Ansprüche, wobei das Fermentationserzeugnis Milchsäure umfasst.

3. Fermentationsverfahren nach einem der vorhergehenden Ansprüche, wobei die hinzuzufügende Enzymmenge basierend auf der Isomaltosemenge in der Gesamtmenge des Stärkehydrolysats und der Aktivität des Enzyms beim Verwenden in einer Fermentationsbrühe berechnet wird, die höchstens 20 g/l Glucose enthält.

4. Fermentationsverfahren nach einem der vorhergehenden Ansprüche, wobei die Glucosekonzentration der Fermentationsbrühe, die einen ersten Anteil einer Gesamtmenge des Stärkehydrolysats von Schritt a) enthält, von etwa 50 g/l bis etwa 150 g/l beträgt.

5. Fermentationsverfahren nach einem der vorhergehenden Ansprüche, wobei das Stärkehydrolysat 85 - 98 Gew.-% Glucose enthält, basierend auf dem Gesamtkohlenhydrat.

6. Fermentationsverfahren nach einem der vorhergehenden Ansprüche, wobei das Stärkehydrolysat 90 - 97 Gew.-% Glucose enthält, basierend auf dem Gesamtkohlenhydrat.

7. Fermentationsverfahren nach einem der vorhergehenden Ansprüche, wobei das Stärkehydrolysat 0,5 - 3 Gew.-% Isomaltose enthält, basierend auf dem Gesamtkohlenhydrat.

8. Fermentationsverfahren nach einem der vorhergehenden Ansprüche, wobei das Stärkehydrolysat 1 - 2 Gew.-% Isomaltose enthält, basierend auf dem Gesamtkohlenhydrat.

9. Fermentationsverfahren nach einem der Ansprüche 1 - 8, wobei ein Echtzeitüberwachungssystem mit einer Anlage verbunden ist, die das Einführen von Stärkehydrolysat in Schritt (d) steuert.

## Revendications

1. Procédé de fermentation discontinue destiné à fermenter un hydrolysat d'amidon contenant 80 à 98 % en poids de glucose par rapport aux glucides totaux et 0,3 à 5 % en poids d'isomaltose par rapport aux glucides totaux en un produit de fermentation, comprenant :
a) la formation d'un bouillon de fermentation contenant une première partie d'une quantité totale de l'hydrolysat d'amidon de telle sorte que le bouillon de fermentation ait une concentration initiale en glucose d'au moins 50 g/L ;
b) la fermentation de l'hydrolysat d'amidon dans le bouillon de fermentation lors d'une étape initiale de fermentation pour produire un produit de fermentation jusqu'à ce que le bouillon de fermentation contienne 30 g/L ou moins de glucose ;
c) l'ajout au bouillon de fermentation d'une quantité efficace d'au moins une enzyme active qui convertit l'isomaltose en glucose ;
d) l'introduction de la quantité restante de la quantité totale d'hydrolysat d'amidon contenant 80 à 98 % en poids de glucose par rapport aux glucides totaux et 0,3 à 5 % en poids d'isomaltose par rapport aux glucides totaux dans le bouillon de fermentation afin de maintenir une concentration en glucose de 5 à 15 g/L dans le bouillon de fermentation pendant toute l'étape d'alimentation ; et
e) la production d'un bouillon de fermentation final contenant le produit de fermentation ;
le produit de fermentation comprenant un acide organique choisi dans le groupe constitué par les acides carboxyliques hydroxyles, les acides dicarboxyliques et les acides tricarboxyliques ; ou
le produit de fermentation étant choisi dans le groupe parmi l'acide lactique, l'acide citrique, l'acide malonique, l'acide hydroxy butyrique, l'acide adipique, la lysine, l'acide cétoglutarique, l'acide glutarique, l'acide 3-hydroxy-propionique, l'acide succinique, l'acide malique, l'acide fumarique, l'acide itaconique, l'acide muconique, l'acide méthacrylique, et l'acide acétique.

2. Procédé de fermentation selon l'une quelconque des revendications précédentes, le produit de fermentation comprenant de l'acide lactique.

3. Procédé de fermentation selon l'une quelconque des revendications précédentes, la quantité d'enzyme à ajouter étant calculée sur la base de la quantité d'isomaltose dans la quantité totale d'hydrolysat d'amidon et de l'activité de l'enzyme lorsqu'elle est utilisée dans un bouillon de fermentation qui contient 20 g/L ou moins de glucose.

4. Procédé de fermentation selon l'une quelconque des revendications précédentes, la concentration en glucose du bouillon de fermentation contenant une première partie d'une quantité totale de l'hydrolysat d'amidon de l'étape a) étant d'environ 50 g/L à environ 150 g/L.

5. Procédé de fermentation selon l'une quelconque des revendications précédentes, l'hydrolysat d'amidon contenant 85 à 98 % en poids de glucose par rapport aux glucides totaux.

6. Procédé de fermentation selon l'une quelconque des revendications précédentes, l'hydrolysat d'amidon contenant 90 à 97 % en poids de glucose par rapport aux glucides totaux.

7. Procédé de fermentation selon l'une quelconque des revendications précédentes, l'hydrolysat d'amidon contenant 0,5 à 3 % en poids d'isomaltose par rapport aux glucides totaux.

8. Procédé de fermentation selon l'une quelconque des revendications précédentes, l'hydrolysat d'amidon contenant 1 à 2 % en poids d'isomaltose par rapport aux glucides totaux.

9. Procédé de fermentation selon l'une quelconque des revendications 1 à 8, un système de surveillance en temps réel s'interfaçant avec un équipement qui régule l'introduction d'hydrolysat d'amidon à l'étape (d).
